# EUROPEAN PATENT APPLICATION

(11) **EP 4 623 888 A1**
(43) Date of publication of application: **01.10.2025**
(21) Application number: 23937630.4
(22) Date of filing: 17.10.2023
(51) Int. Cl.: A61K 8/02, A61K 8/34, A61K 8/55, A61K 8/37, A61K 8/365, A61K 8/44, A61K 8/73, A61K 8/92, A61K 8/891

(54) **METHOD FOR PREPARING MICROCURRENT-FORMING LIQUID CRYSTAL NANOEMULSION**

(30) Priority: 17.05.2023 KR 20230063984; 16.10.2023 KR 20230137766
(71) Applicant: New&New Co., Ltd., Cheonan-si Chungcheongnam-do 31094 (KR)
(72) Inventor: KIM, Hyo Tae, Seoul 06331 (KR); LEE, Chang Eon, Cheonan-si Chungcheongnam-do 31161 (KR); LEE, Ho Young, Asan-si Chungcheongnam-do 31487 (KR)
(74) Representative: RGTH
(86) International application number: PCT/KR2023/016069
(87) International publication number: WO 2024/237400

(57) **Abstract**

Disclosed is a method of preparing a microcurrent-forming liquid crystal nanoemulsion. The microcurrent-forming liquid crystal nanoemulsion can further increase electrical conductivity and form voltages well to generate higher microcurrents, thereby exhibiting beneficial effects of increasing skin absorption of active substances and improving skin elasticity.

## Description

### TECHNICAL FIELD

The present invention relates to a method of preparing a microcurrent-forming liquid crystal nanoemulsion and more specifically, to a method of preparing a liquid crystal nanoemulsion that can exhibit beneficial effects of increasing skin absorption of active substances and improving skin elasticity by further increasing electrical conductivity and forming voltages well to generate higher microcurrents.

### BACKGROUND ART

It has been reported that microcurrents have beneficial functions such as wound healing, increasing cellular activity, improving skin tone, and enhancing skin elasticity. Therefore, the beauty device market using microcurrents has continued to grow steadily in recent years. However, beauty devices have the disadvantages of high price and inconvenience to use and carry. Accordingly, there is a need for development of composition-type cosmetics that can generate microcurrents without a beauty device.

Accordingly, attempts have recently been made to apply the concept of piezoelectricity (a phenomenon in which a microcurrent or voltage is generated by polarization when force is applied) to cosmetics based on the method by which consumers use cosmetics by applying the same to the skin, followed by tapping or rubbing. In other words, a microcurrent generation mechanism in which a microcurrent is formed when pressure is applied during application has been reseached.

However, the application of pressure as described above is cumbersome for the consumer. Therefore, there is a need for a novel method for generating microcurrents without direct application of pressure, effectively delivering active ingredients to the skin layer, increasing absorption thereof into the skin, and providing beneficial functionality to the skin (enhancing elasticity, strengthening the skin barrier, and the like) based on microcurrents.

### DISCLOSURE OF INVENTION

### TECHNICAL PROBLEM

It is an object of the present invention to provide a method of preparing a liquid crystal nanoemulsion that can exhibit beneficial effects of increasing skin absorption of active substances and improving skin elasticity by further increasing electrical conductivity and forming voltages well to generate higher microcurrents.

### TECHNICAL SOLUTION

In accordance with the present invention, provided is a method of preparing a microcurrent-forming liquid crystal nanoemulsion including primarily mixing a phase A with a phase B (a), and secondarily mixing the resulting mixture with a phase C (b), wherein the phase A is a mixture of oil, polyol, phospholipid, and an emulsifier, the phase B is a mixture of purified water, and an acryl- or gum-based thickener that imparts conductivity to a solution in which the thickener is dissolved in purified water, and the phase C is a mixture of purified water, and an organic acid or an organic acid salt that imparts conductivity to a solution in which the organic acid or organic acid salt is dissolved in purified water.

Preferably, a phase D comprising a mixture of purified water and a neutralizer is added to the phase B.

Preferably, an amino acid is further mixed with the phase C.

Preferably, the polyol includes at least one selected from octyldodecanol and glycerol.

Preferably, the phospholipid includes at least one selected from phytosphingosine and hydrogenated lecithin.

Preferably, the emulsifier includes at least one selected from a polyglyceryl-based emulsifier and sorbitan olivate.

### EFFECT OF THE INVENTION

Using the present invention, a nanoemulsion with a liquid crystal lamellar structure can be produced. In addition, the interaction between the nano-sized liquid crystal structure and the electrolyte further increases electrical conductivity and generates better voltage, making it possible to perform the role of a chemical battery to a greater extent.

It is believed that the highly penetrating nano-sized emulsion of the present invention will generate higher microcurrents, thereby maximizing beneficial effects such as increased absorption of effective substances and improved skin elasticity.

### BRIEF DESCRIPTION OF THE DRAWINGS

FIG. 1 is a schematic diagram illustrating a method of preparing a microcurrent-forming liquid crystal nanoemulsion of the present invention;
FIG. 2 is a transmission electron microscopy (TEM) image of Examples;
FIG. 3 is an optical image of Examples;
FIG. 4 shows zeta potentials of Examples;
FIG. 5 shows electrical conductivity of Examples; and
FIG. 6 is an image showing light emission of light-emitting diodes to which Examples are applied.

### MODE FOR CARRYING OUT THE INVENTION

In the present invention, a liquid crystal nanoemulsion was prepared using a thickener and an appropriate combination of a polyol and an organic acid. It was found that much microcurrent can be generated in a formulation without applying a pressure to the skin by increasing electrical conductivity and forming a high voltage to impart the function as a chemical battery to the formulation. In other words, a liquid crystal emulsion having a lamellar structure is formed in nanoscale, thus increasing the overall surface area and the charge potential. Therefore, the charge interaction of the liquid crystal nanoemulsion with an electrolyte becomes higher compared to general emulsions and liquid crystal emulsions.

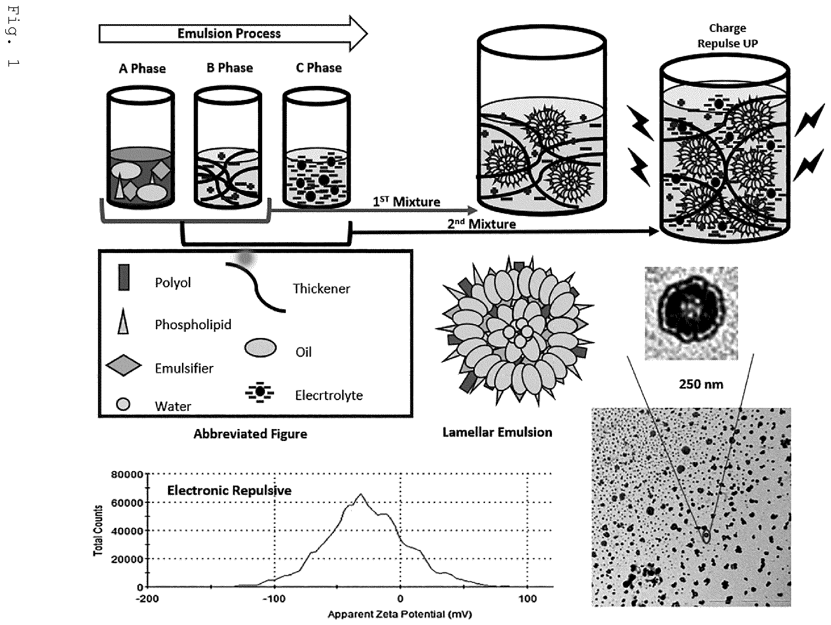

Specifically, the present invention provides a method of preparing a microcurrent-forming liquid crystal nanoemulsion including primarily mixing a phase A with a phase B (a) and secondarily mixing the resulting mixture with a phase C (b), wherein the phase A is a mixture of an oil, a polyol, a phospholipid, and an emulsifier, the phase B is a mixture of purified water and an acryl- or gum-based thickener that imparts conductivity to a solution in which the thickener is dissolved in purified water, and the phase C is a mixture of purified water, and an organic acid or an organic acid salt that imparts conductivity to a solution in which the organic acid or the organic acid salt is dissolved in purified water. FIG. 1 is a schematic diagram illustrating a method of preparing a microcurrent-forming liquid crystal nanoemulsion of the present invention.

The method of preparing the microcurrent-forming liquid crystal nanoemulsion according to the present invention will be described in detail in each step as follows.

### <Step (a): Primary mixing between phase A and phase B>

This step (a) is a process of mixing phase A with phase B. Phase A is a mixture of an oil, a polyol, a phospholipid, and an emulsifier.

Any oil may be used without particular limitation so long as it is widely used in cosmetics. Any oil that may be classified as an oil, such as a fatty acid or an ester oil, may be used. Preferably, the oil is added in an amount of 0.1 to 30% (w/w) with respect to the total composition. Specifically, the oil is for example caprylic/capric triglyceride.

Polyol refers to an organic compound containing a plurality of hydroxyl groups. Representative examples of the polymer include glycerol, dipropylene glycol, pentylene glycol, propylene glycol, diglycerin, propanediol, methyl propanediol, and butylene glycol. Here, preferably, the polymer is added in an amount of 0.1 to 20% (w/w) with respect to the total composition. Any polymer may be used without particular limitation and the polyol may be used singly or as a mixture. For example, the polyol is a mixture of octyldodecanol and glycerol.

A phospholipid is a lipid containing a polar phosphate and a nonpolar fatty acid, and is a major ingredient of biological membranes. Phospholipids are amphipathic and thus allow hydrophobic fatty acid moieties to aggregate in an aqueous solution environment such as water to form stabilized micelles or liposomes. Any phospholipid may be used without particular limitation and the phospholipid may be used singly or as a mixture. Here, the phospholipid is preferably added at 0.01 to 5% (w/w) with respect to the total composition. For example, the phospholipid is a mixture of phytosphingosine and hydrogenated lecithin.

Any emulsifier may be used without particular limitation and the emulsifier may be used singly or as a mixture. The emulsifier is preferably a mixture of sorbitan olivate and a polyglyceryl-based emulsifier. Preferably, the emulsifier is added in an amount of 0.1 to 20% (w/w) with respect to the total composition.

Examples of the polyglyceryl-based emulsifier include polyglyceryl-4 caprate, polyglyceryl-3 polyricinoleate, diisostearoyl polyglyceryl-3 dimer dilinoleate, polyglyceryl-4-diisostearate/polyhydroxystearate/sebacate, polyglyceryl-10 oleate, polyglyceryl-6 polyhydroxystearate, polyglyceryl-6 polyricinoleate, polyglyceryl-3 oleate, polyglyceryl-2 dipolyhydroxystearate, polyglyceryl-3 diisostearate, polyglyceryl-10 dioleate, polyglyceryl-10 decaisostearate, polyglyceryl-10 laurate, polyglyceryl-10 myristate, polyglyceryl-10 stearate, polyglyceryl-2 isostearate/dimer dilinoleate copolymer, polyglyceryl-2 stearate, polyglyceryl-2 triisostearate, polyglyceryl-3 caprate, polyglyceryl-3 methylglucose distearate, polyglyceryl-3 polydimethylsiloxyethyl dimethicone, polyglyceryl-3 stearate, polyglyceryl-4 isostearate, polyglyceryl-5 oleate, polyglyceryl-8 decabehenate/caprate, polyglyceryl-4 oleate, polyglyceryl-6 distearate, polyglyceryl-3 beeswax, poly glyceryl-6 caprylate, polyglyceryl-6 stearate, polyglyceryl-6 behenate, polyglyceryl-10 distearate, polyglyceryl-3 distearate, polyglyceryl-4 laurate, polyglyceryl-6 laurate, polyglyceryl-3 ricinoleate, and polyglyceryl-3 cocoate.

Meanwhile, the phase B is a mixture of purified water and an acryl-based thickener or gum-based thickener that imparts conductivity to a solution in which the thickener is dissolved in purified water. In the present invention, the added thickener causes separation of nanoparticles and improved emulsification as well as the thickening effect. In addition, the acryl-based thickener or gum-based thickener becomes conductive when dissolved in purified water. As a result, the liquid crystal nanoemulsion of the present invention can further increase electrical conductivity and increase the voltage, and can act more effectively as a chemical battery. In the present invention, the thickener is preferably added in an amount of 0.01 to 2.00% (w/w) with respect to the total composition.

Some thickeners require addition of a neutralizer to adjust pH and improve viscosity. Purified water is separately mixed with a neutralizer to prepare a phase D and then the phase D is added to the phase B. Examples of the neutralizer include tromethamine, L-arginine, triethanolamine (TEA), and the like. In the present invention, the neutralizer is preferably added in an amount of 0.01 to 2.00% (w/w) with respect to the total composition.

### <Step (b): Secondary mixing of mixture obtained by primary mixing with phase C>

This step (b) is a process of secondarily mixing a phase C with the resulting mixture obtained by the primary mixing in step (a).

The phase C is a mixture of purified water and an organic acid or organic acid salt that imparts conductivity to a solution in which the organic acid or organic acid salt is dissolved in purified water. When the organic acid or organic acid salt is dissolved in purified water, it dissociates to produce an electrolyte solution. The phase C, which becomes an electrolyte solution in this way, generates a voltage and increases electrical conductivity. In the present invention, the organic acid or organic acid salt is preferably added in an amount of 0.01 to 5% (w/w) with respect to the total composition. Any organic acid or organic acid salt may be used without particular limitation and the organic acid or organic acid salt may be used singly or as a mixture. The organic acid is for example citric acid, and the organic acid salt is for example sodium citrate.

Meanwhile, the phase C is preferably further mixed with amino acid. In the present invention, the amino acid is preferably added in an amount of 0.01 to 5% (w/w) with respect to the total composition. Amino acid is charged and thus further increases electrical conductivity and facilitates formation of a voltage in the liquid crystal nanoemulsion of the present invention. The amino acid is for example betaine. A preservative may be added to the phase C, if necessary. Any preservative may be used without particular limitation and the preservative may be used singly or as a mixture. Examples of the preservative include 1,2-hexanediol and ethylhexylglycerin.

The process of preparing the microcurrent-forming liquid crystal nanoemulsion according to the present invention is described as follows. First, phases A and C were heated and then cooled. Then, phase B (or phase B containing phase D) is added to phase A, followed by primary stirring, preferably, preferably using a homomixer. Then, phase C is added in a portionwise manner, followed by secondary mixing, preferably, using a homomixer.

The liquid crystal nanoemulsion of the present invention prepared by the method has excellent electrical conductivity and can form a microcurrent well, and exhibit galvanic effects of increasing skin absorption through ionization of active substances in the cosmetic composition or effects of improving elasticity and strengthening the skin barrier of the microcurrent. Therefore, the liquid crystal nanoemulsion prepared by the method of the present invention may be added to various formulations of cosmetic compositions or cosmetics.

Hereinafter, the present invention will be described in more detail with reference to the following examples and experimental examples, but the scope of the present invention is not limited to the examples and experimental examples, and includes variations and technical concepts equivalent thereto.

### [Example: Preparation of microcurrent-forming liquid crystal nanoemulsion according to present invention]

In this example, various thickeners were prepared based on an oil and the microcurrent-forming abilities thereof were compared. Caprylic/capric triglyceride, which is commonly used in cosmetics, was selected as the oil. The thickener was selected from acrylic thickeners and natural gums that provide particle emulsion stability and can trap particles.

In Table 1 below, the phase A is an oil phase to which the emulsifier has been added. Caprylic/capric triglyceride was used as the oil, and octyl dodecanol and glycerol were used as polyols. Sorbitan olivate and polyglyceryl-4 laurate, which is a polyglyceryl-based emulsifier, were used as emulsifiers. Phytosphingosine and hydrogenated lecithin were used as phospholipids.

The phase B is a thickener phase. In this example, various thickeners were prepared in various ways in respective Examples, as shown in Table 1 below, and were dissolved in purified water prior to use.

The phase C is an aqueous phase. The phase C contains an organic acid or organic acid salt that imparts conductivity to a solution in which the organic acid or organic acid salt is dissolved in purified water. Citric acid was used as the organic acid and sodium citrate was used as the organic acid salt. In addition, betaine was added as an amino acid and a preservative was also added.

The phase D is an aqueous phase in the form of an aqueous solution to which the neutralizer has been added. When acrylates/C10-30 alkyl and polyacylic acid were used as thickeners, the phase D was added to the thickener. Tromethamine was used as a neutralizer.

The process of preparing the microcurrent-forming liquid crystal nanoemulsion according to the present invention is described as follows. First, phases A and C were heated (at 85°C) and then cooled (at 30°C or less). Then, the phase B or the phase B containing the phase D was added to the phase A, followed by stirring with a homomixer for about 5 to about 10 minutes. Then, the phase C was added in a small amount, followed by stirring with a homomixer for about 10 minutes.

**[Table 1]**

| Compositions of Examples 1 to 9 | | | | | | | | | | | | |
|---|---|---|---|---|---|---|---|---|---|---|---|---|
| No . | Examples | | | | | | | | | | | |
| | | | | Exa mpl e 1 | Exa mpl e 2 | Exa mpl e 3 | Exa mpl e 4 | Exa mpl e 5 | Exa mpl e 6 | Exa mpl e 7 | Exa mpl e 8 | Exa mpl e 9 |
| 1 | A | Oil | Capryli c/Capri c triglyc eride | 1.5 | 1.5 | 1.5 | 1.5 | 1.5 | 1.5 | 1.5 | 1.5 | 1.5 |
| 2 | A | Polyol | Octyldo decanol | 3 | 3 | 3 | 3 | 3 | 3 | 3 | 3 | 3 |
| 3 | A | Emulsi fier | Sorbita n Olivate | 2 | 2 | 2 | 2 | 2 | 2 | 2 | 2 | 2 |
| 4 | A | Polyol | Glycero l | 15 | 15 | 15 | 15 | 15 | 15 | 15 | 15 | 15 |
| 5 | A | Phosph olipid | Phytosp hingosi ne | 0.0 5 | 0.0 5 | 0.0 5 | 0.0 5 | 0.0 5 | 0.0 5 | 0.0 5 | 0.0 5 | 0.0 5 |
| 6 | A | Phosph olipid | Hydroge nated Lecithi n | 0.5 | 0.5 | 0.5 | 0.5 | 0.5 | 0.5 | 0.5 | 0.5 | 0.5 |
| 7 | A | Emulsi fier | Polygly ceryl-4 Laurate | 0.5 | 0.5 | 0.5 | 0.5 | 0.5 | 0.5 | 0.5 | 0.5 | 0.5 |
| 8 | B | Acryli c thicke ner | Sodium polyacy late | 0.2 | | | | | | | | |
| 9 | B | Acryli c thicke ner | Polyacr ylamide | | 0.2 | | | | | | | |
| 10 | B | | Water | 10 | 10 | 10 | 10 | 10 | 10 | 10 | 10 | 10 |
| 11 | B | Acryli c thicke ner | Acrylat es/C10-30 Alkyl Acrylat e Crosspo lymer | | | 0.2 | | | | | | |
| 12 | B | Acryli c thicke ner | Polyacr ylic acid | | | | 0.2 | | | | | |
| 13 | B | Acryli c thicke ner | Ammoniu m acryloy ldimeth yltaura te/VP copolym er | | | | | 0.2 | | | | |
| 14 | B | Gum-based thicke ner | Xanthan Gum | | | | | | 0.2 | | | |
| 15 | B | Gum-based thicke ner | Diutan Gum | | | | | | | 0.2 | | |
| 16 | B | Gum-based thicke ner | Ethylhe xyl cellulo se | | | | | | | | 0.2 | |
| 17 | B | Gum-based thicke ner | Acasia gum | | | | | | | | | 0.2 |
| 18 | D | | Water | | | 3 | 3 | | | | | |
| 19 | D | Neutra lizer | Trometh amine | | | 0.1 6 | 0.1 6 | | | | | |
| 20 | C | | Water | to1 00 | to1 00 | to1 00 | to1 00 | to1 00 | to1 00 | to1 00 | to1 00 | to1 00 |
| 21 | C | Preser vative | 1,2-Hexaned iol | 1.5 | 1.5 | 1.5 | 1.5 | 1.5 | 1.5 | 1.5 | 1.5 | 1.5 |
| 22 | C | Preser vative | Ethylhe xylglyc erine | 0.0 5 | 0.0 5 | 0.0 5 | 0.0 5 | 0.0 5 | 0.0 5 | 0.0 5 | 0.0 5 | 0.0 5 |
| 23 | C | Amino acid | Betaine | 2 | 2 | 2 | 2 | 2 | 2 | 2 | 2 | 2 |
| 24 | C | Organi c acid | Citric acid | 0.1 | 0.1 | 0.1 | 0.1 | 0.1 | 0.1 | 0.1 | 0.1 | 0.1 |
| 25 | C | Organi c acid salt | Sodium citrate | 0.0 5 | 0.0 5 | 0.0 5 | 0.0 5 | 0.0 5 | 0.0 5 | 0.0 5 | 0.0 5 | 0.0 5 |

### [Experimental Example 1: Transmission electron microscopy of Examples]

The overall distributions of Examples prepared above were observed in terms of morphology using a transmission electron microscope (TEM), to determine whether or not liquid crystal lamellar structures and nano-sized emulsions were well formed.

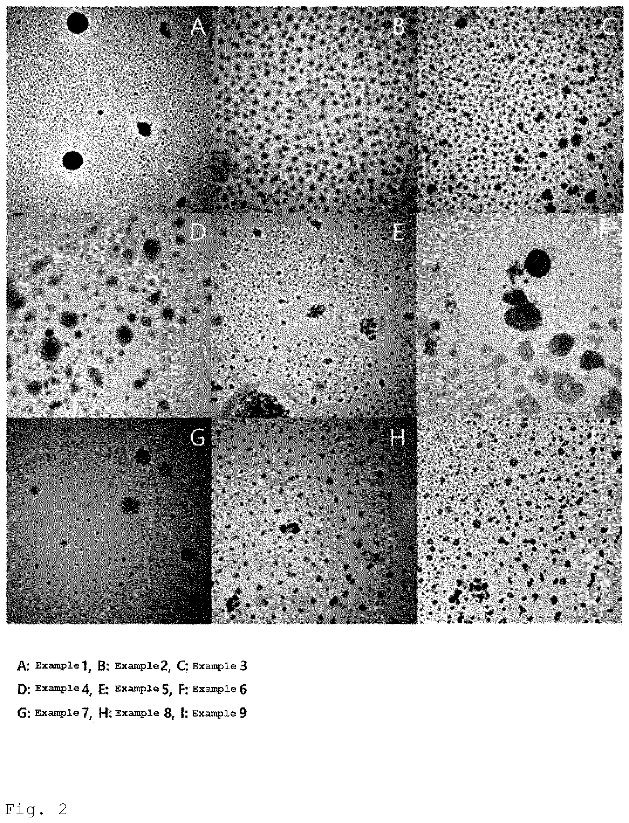

The results are shown in FIG. 2. Overall, it can be seen that lamellar membrane structures and particles of about 2 to 300 nm in size were evenly distributed in all Examples.

### [Experimental Example 2: Polarizing microscopy of Examples]

Whether or not brightly shining lamellar multilayer structures were formed in compositions of Examples was observed using a polarizing microscope among optical microscopes.

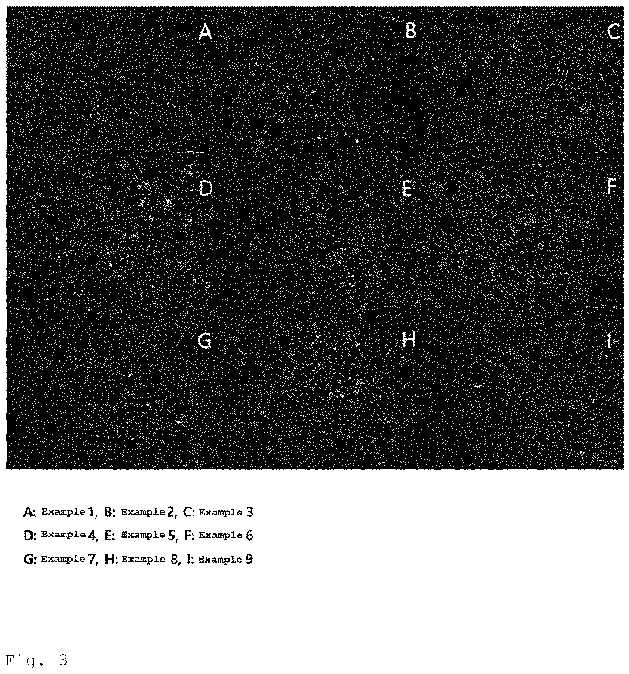

The results are shown in FIG. 3. Brightly shining crystal-shaped particles having multi-layer lamellar structures were observed in compositions of all examples.

### [Experimental Example 3: Confirmation of electrostatic potential of Examples]

Zeta potential of Examples was measured to detect the electrostatic potential.

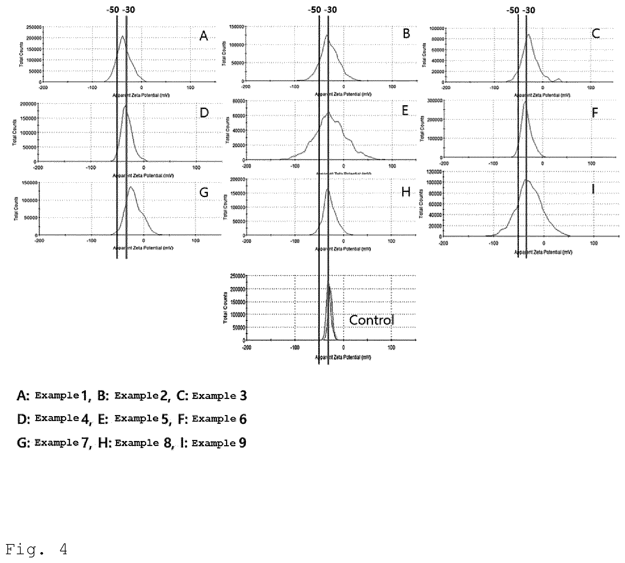

The experimental results are shown in FIG. 4. The results show that, in all examples, a zeta potential of -30 or less was recorded and zeta potentials for respective particles of -50 or less were recorded. On the other hand, the zeta potential of a conventional galvanic cream (Korean Patent No. 10-2559008) as a control was measured. The result showed that the zeta potential of the conventional galvanic cream was -30 or less, but was not greater than -50.

Typically, when the absolute value of the zeta potential is -30 or less, the repulsive force of the zeta potential is referred to as "stable", and when the absolute value of the zeta potential is -50 or less, the repulsive force of the zeta potential is referred to as "higher electrostatic repulsive force".

The zeta potential of the conventional galvanic cream (Korean Patent No. 10-2559008) did not exceed -50, whereas the zeta potentials of all examples according to the present invention exceeded -50. In particular, the zeta potentials in Examples 5 and 9 reached -100.

Accordingly, all Examples had excellent electrostatic repulsion, in particular, Examples 5 and 9 were found to be the best.

### [Experimental Example 4: Current and voltage measurements of Examples]

In order to compare the actual current and voltage, the currents and voltages of Examples and controls were measured and compared (Table 2). Control 1 is the conventional galvanic cream (Korea Patent No. 10-2559008), whereas Control 2 is a galvanic product from another company.

**[Table 2]**

| Results of current and voltage measurement | | | | | | |
|---|---|---|---|---|---|---|
| Example | Current (µA) | | | Voltage (V) | | |
| 1 | 0.667 | ± | 0.047 | 2.667 | ± | 0.471 |
| 2 | 0.733 | ± | 0.047 | 3.000 | ± | 0.816 |
| 3 | 0.467 | ± | 0.047 | 4.000 | ± | 0.816 |
| 4 | 0.700 | ± | 0.082 | 3.667 | ± | 0.471 |
| 5 | 0.833 | ± | 0.047 | 4.333 | ± | 0.943 |
| 6 | 0.533 | ± | 0.082 | 3.000 | ± | 0.000 |
| 7 | 0.467 | ± | 0.047 | 2.000 | ± | 0.000 |
| 8 | 0.367 | ± | 0.047 | 2.333 | ± | 0.471 |
| 9 | 0.700 | ± | 0.047 | 4.667 | ± | 0.471 |
| Control 1 | 0.039 | ± | 0.001 | 0.008 | ± | 0.001 |
| Control 2 | 0.002 | ± | 0.000 | 0.135 | ± | 0.034 |

As can be seen from Table 2, both the current and voltage of Examples were much higher than those of Control 1 and Control 2. In particular, Examples 5 and 9 were found to be excellent.

### [Experimental Example 5: Measurement of skin electrical conductivity of Examples]

To measure the skin electrical conductivity of Examples, the electrical conductivity was measured after application to the skin. The electrical conductivities of distilled water, Control (general cream), and Control 2 (galvanic cream of Korea Patent No. 10-2559008) were also measured.

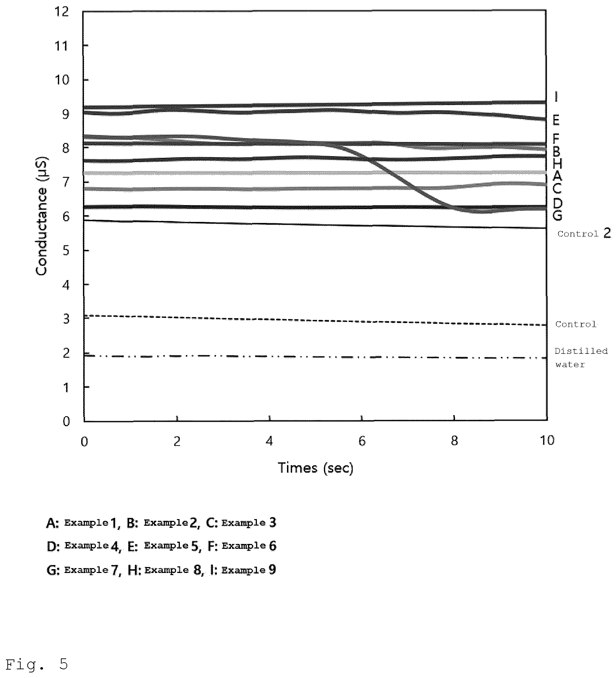

The experimental results are shown in FIG. 5. The results show that all Examples had higher electrical conductivity than the controls, and in particular, Examples 5 and 9 exhibited the highest electrical conductivity.

### [Experimental Example 6: Measurement of light emission of light-emitting diodes using Examples]

Whether or not current was generated in light-emitting diodes, to which Examples were applied, and the light-emitting diodes emit light was determined.

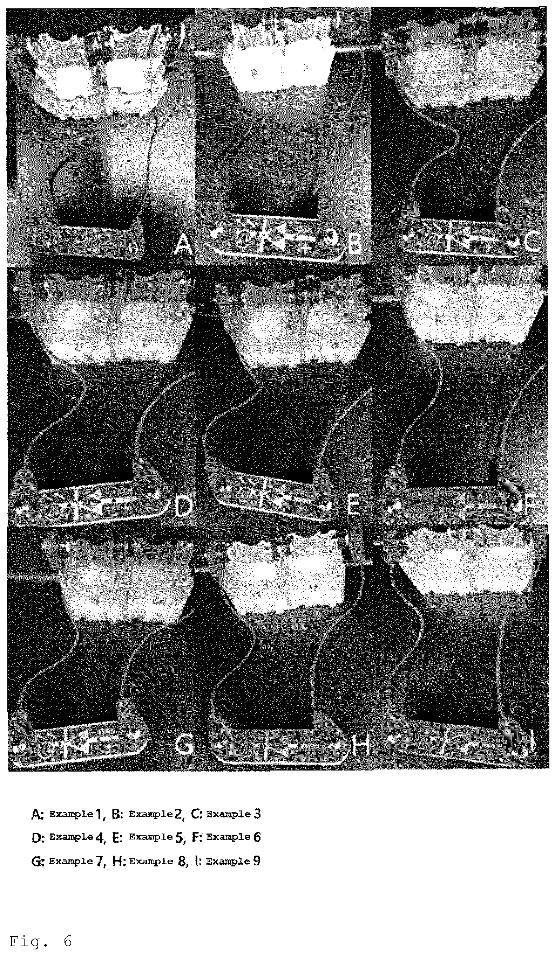

The experimental results are shown in FIG. 6. The light-emitting diodes, to which all Examples were applied, emitted red light (bulbs of the light-emitting diodes emit red light).

## Claims

1. A method of preparing a microcurrent-forming liquid crystal nanoemulsion comprising:
primarily mixing a phase A with a phase B (a); and
secondarily mixing the resulting mixture with a phase C (b),
wherein the phase A is a mixture of oil, polyol, phospholipid, and an emulsifier,
the phase B is a mixture of purified water, and an acryl- or gum-based thickener that imparts conductivity to a solution in which the thickener is dissolved in purified water, and
the phase C is a mixture of purified water, and an organic acid or an organic acid salt that imparts conductivity to a solution in which the organic acid or organic acid salt is dissolved in purified water.

2. The method according to claim 1, wherein a phase D comprising a mixture of purified water and a neutralizer is added to the phase B.

3. The method according to claim 1, wherein an amino acid is further mixed with the phase C.

4. The method according to claim 1, wherein the polyol comprises at least one selected from octyldodecanol and glycerol.

5. The method according to claim 1, wherein the phospholipid comprises at least one selected from phytosphingosine and hydrogenated lecithin.

6. The method according to claim 1, wherein the emulsifier comprises at least one selected from a polyglyceryl-based emulsifier and sorbitan olivate.
